# EUROPÄISCHE PATENTSCHRIFT

(11) **EP 1 496 112 B1**
(45) Veröffentlichungstag und Bekanntmachung des Hinweises auf die Patenterteilung: **23.06.2010**
(21) Anmeldenummer: 04012321.8
(22) Anmeldetag: 25.05.2004
(51) Int. Cl.: C12N 9/50

(54) **Verfahren zur Isolierung von Inselzellen aus Pankreasgewebe**
Process for isolation of islet cells from pancreatic tissue
Procédé d'isolement de cellules insulaires à partir de tissu pancréatique

(30) Priorität: 09.07.2003 DE 10331171
(43) Veröffentlichungstag der Anmeldung: 12.01.2005
(73) Patentinhaber: Nordmark Arzneimittel GmbH & Co.KG, 25436 Uetersen (DE)
(72) Erfinder: Kurfürst, Manfred, Dr., 25436 Moorege (DE); Raemsch, Christian, Dr., 25436 Uetersen (DE); Raemsch-Guenter, Nicole, Dr., 25436 Uetersen (DE); Friedrich, Olaf, Dr., 25436 Tomesch (DE); Huettler, Silke, Dr., 25436 Uetersen (DE); Brandhorst, Daniel, Dr., 35440 Linden (DE); Berney, Thierry, Dr., 74800 Arenthon (FR); Bucher, Pascal, Dr., 1255 Veyrier-Geneva (CH)
(74) Vertreter: Richter, Werdermann, Gerbaulet & Hofmann

(56) Entgegenhaltungen:
- WO-A-96/19583
- WO-A-03/004628
- HEFLEY T J ET AL: "ENZYMATIC ISOLATION OF CELLS FROM NEONATAL CALVARIA USING TWO PURIFIED ENZYMES FROM CLOSTRIDIUM HISTOLYTICUM" EXPERIMENTAL CELL RESEARCH, SAN DIEGO, CA, US, Bd. 149, Januar 1983 (1983-01), Seiten 227-236, XP002000526 ISSN: 0014-4827

## Beschreibung

Die Erfindung betrifft ein Verfahren zur Isolierung von Inselzellen aus Pankreasgewebe und die Verwendung von neutraler Protease.

Die DE 44 45 891 A1 beschreibt eine rekombinante Proteinase (neutrale Protease, NP) aus Clostridium histolyticum und ihre Verwendung zur Isolierung von Zellen und Zellverbänden. Es wird dabei davon ausgegangen, dass, um neutrale Protease zur Isolierung von Zellen und Zellverbänden in großem Umfang einsetzen zu können, es erforderlich ist, die neutrale Protease in reproduzierbarer Qualität und in großen Mengen zur Verfügung zu stellen, was durch ein rekombinantes Herstellungsverfahren möglich ist. Die Klonierung der neutralen Protease ist jedoch nicht ohne weiteres möglich, da kaum Sequenzen aus Clostridium histolyticum bekannt sind. Damit sind auch Schlüsse auf die Codon usage und damit die Einengung der Degeneriertheit von verwendbaren Oligonukleotiden nicht möglich.

Der US 5,989,888 ist zu entnehmen, ein vom Hersteller hergestelltes fertiges Produkt, aus einer Mischung aus bestimmten Verhältnissen von Kollagenasen I und II und neutraler Protease.

Nach diesem Verfahren wird eine neutrale Protease eingesetzt, die in einer Kollagenase-Präparation enthalten ist, wodurch keine große Stabilität der Enzyme erreicht wird. Hinzukommt, dass, wenn die neutrale Protease in der Kollagenase längere Zeit enthalten ist, dann bei Feuchtigkeit eine Enzymaktivitätsabnahme eintritt.

Aus Hefley et al. "Enzymatic Isolation of Cells from Neonatal Calvaria Using Two Purified Enzymes from *Clostridium histolyticum"* ist die gemeinsame Verwendung von Kollagenase und neutraler Protease bekannt. Beide Enzyme werden aus einer Kollagenase-Enzympräparation von *Clostridium histolyticum* gewonnen.

Es ist Aufgabe der vorliegenden Erfindung, ein Verfahren zur Isolierung von Inselzellen aus Pankreasgewebe bereitzustellen, das verbesserte Isolierungsergebnisse im Hinblick auf die Ausbeute, Viabilität und Unversehrtheit der Zellen erreicht.

Gelöst wird diese Aufgabe durch ein Verfahren gemäß Anspruch 1. Das erfindgemäße Verfahren zur Isolierung von Inselzellen aus Pankreasgewebe umfasst folgende Schritte:
(a) Bereitstellen (i) einer separaten Komponente A aus einer nicht in einer Kollagenase-Enzympräparation enthaltenen Protease (NP) aus *Clostridium histolyticum* in einer in einem Bereich von 4 bis 8 mg/g Pankreasgewebe liegenden Menge und (ii) einer separaten Komponente B aus Kollagenase bzw. aus einer Kollagenase-Enzympräparation in einer in einem Bereich von 0,2 bis 0,6 DMC-U/g Pankreasgewebe liegenden Menge;
(b) Mischen der Komponente A und der Komponente B unmittelbar vor der Zugabe zu dem Pankreasgewebe, wobei das Verhältnis der Komponente A zu Komponente B in Abhängigkeit von der Art der Spezies, aus der das Pankreasgewebe stammt, dem Zustand des Pankreasgewebes, den Lagerungsbedingungen des Pankreasgewebes und/oder der Menge des Pankreasgewebes bestimmt wird, wobei der Gewichtsanteil von Komponente A an der erhaltenen Mischung 3, 6, 12 oder 24 % beträgt;
(c) Zugeben der gemäß Schritt (b) zubereiteten Mischung zu dem Pankreasgewebe; und
(d) Isolation der Inselzellen.

Vorteilhafte Ausgestaltungen der Erfindung sind Gegenstand der Unteransprüche.

Ferner ist ein Verfahren zur Herstellung eines Produktes aus neutraler Protease (NP) und einer Kollagenase für die Isolierung von Inselzellen aus Pankreasgewebe vorgesehen. Das Verfahren umfasst die Schritte:
(a) Bereitstellen (i) einer separaten Komponente A aus einer nicht in einer Kollagenase¬-Enzympräparation enthaltenen Protease (NP) aus *Clostridium histolyticum* in einer in einem Bereich von 4 bis 8 mg/g Pankreasgewebe liegenden Menge und (ii) einer separaten Komponente B aus Kollagenase bzw. aus einer Kollagenase-Enzympräparation in einer in einem Bereich von 0,2 bis 0,6 DMC-U/g Pankreasgewebe liegenden Menge; und
(b) Mischen der Komponente A und der Komponente B unmittelbar vor der Zugabe zu dem Pankreasgewebe, wobei das Verhältnis der Komponente A zu Komponente B in Abhängigkeit von der Art der Spezies, aus der das Pankreasgewebe stammt, dem Zustand des Pankreasgewebes, den Lagerungsbedingungen des Pankreasgewebes und/oder der Menge des Pankreasgewebes bestimmt wird, wobei der Gewichtsanteil von Komponente A an der erhaltenen Mischung 3, 6, 12 oder 24 % beträgt.

Das verfahrensgemäße Produkt besteht aus einer separaten Komponente A aus einer nicht in einer Kollagenase-Enzympräparation enthaltenen Protease (NP) aus Clostridium histolyticum und einer separaten Komponente B aus Kollagenase bzw. aus einer Kollagenase-Enzympräparation, wobei für eine Gewebedissoziation die Komponente A der Komponente B in jeweils erforderlichen Mengen vor dem Einleiten der Dissoziation zugegeben wird.

Des Weiteren umfasst die Erfindung die Verwendung des verfahrensgemäß hergestellten Produktes aus neutraler Protease (NP) und einer Kollagenase für die Isolierung von Inselzellen aus Pankreasgewebe zur Verbesserung der Isolierungsergebnisse im Hinblick auf Ausbeute, Viabilität und Unversehrtheit der Zellen.

Die eingesetzte neutrale Protease wird nicht durch eine rekombinante Herstellung zur Verfügung gestellt.

Erfindungsgemäß ist vorgesehen, dass man eine separate Komponente A aus einer nicht in einer Kollagenase-Enzympräparation enthaltenen, nicht durch eine rekombinante Herstellung erzeugte neutrale Protease (NP) aus Clostridium histolyticum mit einem definierten Gehalt mit einer separaten Komponente B aus einer gereinigten oder angereinigten Kollagenase bzw. aus einer Kollagenase-Enzympräparation bei individueller Dosierung der Mengenverhältnisse der beiden Komponenten vor dem Einleiten der Gewebedissoziation vermischt.

Die erfindungsgemäße Verwendung besteht in einer aufgereinigten nicht in einer Kollagenase-Enzympräparation enthaltenen neutralen Protease (NP) in einer jeweils ausgewählten Menge als individueller Zusatz zu einer Kollagenase bzw. Kollagenase-Enzympräparationen zur Gewebedissoziation bzw. Isolierung von Zellen oder Zellaggregaten, beispielsweise aus Geweben, zur Verbesserung der Isolierungsergebnisse im Hinblick auf Ausbeute, Viabilität und Unversehrtheit der Zellen.

Besonders vorteilhaft ist die Verwendung von neutraler Protease zusammen mit gereinigter oder angereinigter Kollagenase mit definiertem Gehalt an neutraler Protease bei individueller Dosierung der Mengenverhältnisse der beiden Komponenten, wobei neutrale Protease eingesetzt wird, die nicht durch eine rekombinante Herstellung erzeugt wird.

Aufgrund der erfindungsgemäßen Verwendung von neutraler Protease für eine Gewebedissoziation wird eine Verbesserung der Isolierungsergebnisse im Hinblick auf Ausbeute, Viabilität und Unversehrtheit der Zellen, dadurch erreicht, dass neutrale Protease (NP) von Clostridium histolyticum separat, nicht in Kollagenase-Enzympräparation enthalten, eingesetzt wird und zwar durch individuelle Zugabe der neutralen Protease zu einer Kollagenase-Enzympräparation oder Kollagenase oder Kollagenase I und II zur Isolierung von Zellen oder Zellaggregaten aus Geweben. Eingesetzt wird neutrale Protease von Clostridium histolyticum. Dabei besteht Individualität in Abhängigkeit vom Gewebetyp und/oder vom Zustand. Dabei wird so verfahren, dass die Zugabe von Kollagenase und neutraler Protease jeweils in Abhängigkeit von der Gewebemenge erfolgt. Die neutrale Protease ist nicht in einer Kollagenase-Präparation enthalten, wodurch eine größere Stabilität der Enzyme erreicht wird. Hinzukommt, dass keine von einem Hersteller angefertigte und auf Lager oder vorrätig gehaltene Mischung von neutraler Protease und Kollagenase eingesetzt wird. Kurz vor dem Einleiten einer Gewebedissoziation werden neutrale Protease und Kollagenase vereinigt.

Besonders vorteilhaft ist die Demonstration der Funktion beim Verdauen in Abhängigkeit von Spezies, Zustand des Organs und der Lagerung des Organs. Überraschenderweise hat sich gezeigt, dass allein durch die Variation der Zugabe von neutraler Protease die Isolierungsergebnisse signifikant verbessert werden konnten im Hinblick auf Ausbeute an Inselzell-Äquivalenten, Viabilität, Unversehrtheit der Inselzellen bzw. Inselzellaggregate. Dabei spielt die Zugabe an Kollagenase eine weniger kritische Rolle, da es ein für die Zellen relativ wenig toxisches Enzym ist. Die Menge an neutraler Protease ist kritischer, da das Enzym in zu großen Mengen zellschädigend wirkt, in zu geringen Mengen die Gewebedissoziation unvollständig bleibt, also Ausbeute an isolierten Inselzellen und Inselzellaggregaten geringer ist. Um die neutrale Protease individuell dosieren zu können, ist es auch wichtig, dass eine gereinigte oder angereinigte Kollagenase verwendet wird, die geringe Mengen an anderen proteolytischen Aktivitäten wie die der neutralen Protease enthält.

Vorteilhafte Ausgestaltungen der Erfindung sind Gegenstand der Unteransprüche.

Optimierung der Inselisolierung aus einem porzinen und humanen Pankreas durch spenderorientierte Enzymadaption.

Die Isolierung Langerhansscher Inseln aus dem Pankreasgewebe nimmt auf dem Gebiet der enzymatischen Zellseparation eine Sonderstellung ein. Im Gegensatz zur Isolierung anderer Zelltypen aus unterschiedlichen Geweben ist die Inselisolierung gekennzeichnet durch das Bestreben ein vielzelliges Zellagglomerat, die Insel, aus einem anderen Organ, dem Pankreas, unter Präservierung der morphologischen Integrität zu separieren. Diese Zielsetzung stellt an die angewendete Methodik besondere Anforderungen.

Als Standardprozedur zur Isolierung von Inseln aus dem Pankreas von Großtieren hat sich die semiautomatische Digestions-Filtrationsmethode etabliert, die eine enzymatische Verdauung mit einer mechanischen Dissoziation des Pankreas kombiniert. Eine besondere Herausforderung stellt dabei die Selektion geeigneter kollagenolytischer Enzyme bzw. Proteasengemische dar. Diese sollten im Idealfall eine maximale Dissoziation des exokrinen Gewebes bei minimaler Dissoziation des Inselgewebes gewährleisten. Unter der Vielzahl der in unreinen kollagenolytischen Proteasengemischen vorhandenen Enzyme wurden bisher Kollagenasen der Klasse I und Klasse II als essentiell für die Inselfreisetzung aus dem Pankreas von Ratten und Schweinen identifiziert. Da reine Kollagenasen nur zu einer unzureichenden Digestion des Pankreasgewebes von Hund und Ratte führen, scheinen weitere Proteasen in Form von Dispase, neutraler Protease oder Trypsin für eine effiziente Freisetzung von Inseln notwendig zu sein. Eigene präliminäre Befunde am Schweinepankreas sprechen ebenfalls für einen positiv synergistischen Effekt von unspezifischer "Neutraler Protease" auf die Inselfreisetzung. Der Wirkmechanismus von unspezifischen Proteasen liegt hierbei möglicherweise in der Freilegung von Kollagenfasern durch den proteolytischen Abbau von Proteoglykanen, so dass kollagenolytische Aktivitäten einen erleichterten Zugang zu ihren Substraten erhalten. Darüberhinaus wird eine verstärkte Hydrolyse von Kollagenbruchstücken durch unspezifische Protease diskutiert. Ein Überschuss an neutraler Protease kann allerdings den proteolytischen Abbau von Kollagenase selbst beschleunigen und durch die Lyse von Protease sensitiven Adhäsionsmolekülen (CAMs) zur Inseldesintegration beitragen. Auf der anderen Seite konnte an Ratten gezeigt werden, dass Inseln gegenüber der proteolytischen Wirkung von reiner Kollagenase relativ resistent zu sein scheinen.

Bei der Bewertung der zitierten Untersuchungen muss berücksichtigt werden, dass die Sensitivität von Inseln gegenüber der destruktiven Wirkung von neutralen Proteasen speziesabhängig ist und durch das morphologische Verteilungsmuster der Zellkontakte und der Ausprägung der Kollagenmatrix determiniert wird. So ist beim Schwein der periinsuläre Kollagengehalt von allen bisher untersuchten Spezies (Hund, Mensch, Ratte, Rind) am niedrigsten und die Ausprägung einer protektiven Bindegewebskapsel nur rudimentär vorhanden. Dagegen findet man bei dieser Spezies die höchste Anzahl von Zellkontakten zwischen endokrinen und exokrinen Pankreaszellen. Somit sind die Anforderungen, die hinsichtlich des Aktivitätsprofils an ein kollagenolytisches Proteasengemisch gestellt werden, abhängig von der verwendeten Spenderspezies. Diese wichtigen Befunde führten zur Entwicklung und kommerziellen Verwertung von Enzymgemischen für die Inselisolierung aus dem Pankreas der Ratte (Liberase RI^{©}), des Hundes (Liberase^{©}), des Schweines (Liberase PI^{©}) und des Menschen (Liberase HI^{©}) durch die Firma Roche. Diese Produkte unterscheiden sich vor allem im Gehalt an neutraler Protease voneinander.

Neben speziesbedingten Differenzen existieren natürlich auch intraspezifische und individuelle Spendervariabilitäten. Beim menschlichen Pankreasspender induziert vor allem eine altersbedingte Ausprägung von Faktoren wie Ernährungsstatus, Konsumgewohnheiten und Vorerkrankungen eine enorme Varianz der erzielten Ausbeute nach Inselisolierung. Wie auch beim Schwein ist als hochsignifikante Variable für eine isolierbare Inselmasse vor allem das Spenderalter zu nennen, was junge Spender < 25 Jahre als weitgehend ungeeignet für eine erfolgreiche Inselisolierung erscheinen lässt. Altersbedingte Veränderungen, wie ein erhöhter Body Mass Index, die häufig mit einer pankreatischen Fibrotisierung oder Pankreasverfettung assoziiert sind, erhöhen die Isolierbarkeit von Inseln aus dem humanen Pankreas. Obwohl diese morphologischen Veränderungen sogar das Pankreas von geriatrischen Spendern für die Inselisolierung als geeignet erscheinen lassen, ist dabei aber auch ein altersbedingtes Nachlassen der Inselfunktionalität zu berücksichtigen . Daher würde gerade eine Erweiterung des Spenderpools um juvenile bzw. junge Spender das Potential für die klinische Inseltransplantation enorm vergrößern.

Auch beim Schweinepankreas wurde auf die histologischen Unterschiede zwischen adulten und juvenilen Tieren hingewiesen, die eine erfolgreiche Inselisolierung aus dem juvenilen Pankreas enorm erschweren. Neuere Untersuchungen zeigen zudem, dass auch in definierten Zuchtlinien signifikante Unterschiede zwischen Individuen gleichen Alters hinsichtlich der isolierbaren Inselmasse nachweisbar sind.

Die vorausgegangenen Ausführungen führen zu dem Schluss, dass die Ausprägung unterschiedlicher Spenderfaktoren eine individuelle Berücksichtigung bei der Behandlung eines Spenderpankreas erfordern. Eine maßgeschneiderte Isolierungsprozedur beinhaltet unter Berücksichtigung der oben diskutierten histologischen Befunde vor allem eine Modifikation der verwendeten kollagenolytischen Proteasengemische. Verfügbare Enzyme oder Enzymgemische müssten dem Anwender dabei größtmögliche Flexibilität ermöglichen ohne ihn jedoch in der Komplexität eines individuellen Blendings zu überfordern. Die Verfügbarkeit einer reinen Klasse-I/Klasse-II Kollagenasemischung, wie sie in Form der NB-1 Kollagenase der Firma Nordmark/Serva erhältlich ist, kommt dieser Forderung weitgehend entgegen. Abhängig vom jeweiligen Spenderstatus sollte eine Kollagenasekonzentration eingestellt werden, die jeweils durch neutrale Protease individuell ergänzt werden müsste.

Die Erfindung wird durch folgende Ausführungen und Beispiele erläutert:

### Beispiel I

Anpassung der neutralen Proteaseaktivität fördert erfolgreich die Isolierung von Inselzellen vom Schwein nach kalter Langzeitlagerung.

Um die knappen Ressourcen menschlicher Organe weitestgehend zu schonen, werden Vorversuche mit Schweinen durchgeführt. Hierzu zählt vor allem die Titration von Grenzkonzentrationen an Kollagenase und neutraler Protease, die zunächst an adulten (> 24 Monate) Schweinepankreata evaluiert wird. Da die NB-1 Kollagenase zunächst nur in begrenzter Menge zur Verfügung steht, werden Isolierungen von Schweineinseln mit NB-8 Kollagenasen durchgeführt. Hierbei kommen nur Chargen zur Anwendung, die durch einen minimalen Gehalt an neutraler Protease und Trypsin sowie durch einen möglichst geringen Gehalt an Clostripain charakterisiert sind. Vorversuche dazu zeigen, dass die Digestionszeit und die Menge des verdaubaren Gewebes deutlich mit der Konzentration an neutraler Protease korreliert.

Um eine konstante Enzymkonzentration für jede Versuchsreihe zu gewährleisten, wird die distendierte Kollagenasemenge an die Menge des eingesetzten Gewebes angepasst. Dies steht im Kontrast zum propagierten Liberase-Protokoll der Firma Roche, bei dem unabhängig von der Gewebemasse eine konstante Enzymmenge von 500 mg verwendet werden soll. Bei drei zuvor empirisch zu ermittelnden KollagenaseKonzentrationen (z. B. 4 mg/g Organ, 6 mg/g und 8 mg/g) wird die optimale Menge an neutraler Protease im Bereich von ca. 0,2 - 0,6 DMC-U/g Organ iterativ titriert:

| | Neutr. Protease: | NB-8 Kollagenase: | | |
|---|---|---|---|---|
| | | 4 mg/g | 6 mg/g | 8 mg/g |
| 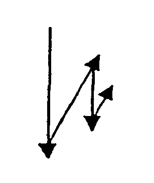 | 0.2 DMC-U/g | | | |
| | 0.3 DMC-U/g | | | |
| | 0.4 DMC-U/g | | | |
| | 0.5 DMC-U/g | | | |
| | 0.6 DMC-U/g | | | |

Als relevante Isolierungsparameter werden die Digestionsdauer, das Verhältnis von eingesetztem zu verdautem Gewebe, die Inselausbeute, die Reinheit und die durchschnittliche Inselgröße erfasst. Die effektivste Mischung wird an 6 Organen getestet. Die dabei isolierten Inseln werden einer Qualitätskontrolle unterzogen, die die Funktionalität und Viabilität der Inseln sowohl in vitro durch statische Glukosestimulation und Membranintegritätsfärbungen als auch in vivo mittels des diabetischen Nacktmaus-Bioassays erfasst. Die vermutlich beste Kombination aus NB-8-Kollagenase und neutraler Protease soll durch Versuche mit NB-1 Kollagenase ebenfalls an 6 Organen bestätigt werden.

Durch empirisch gesammelte Erfahrungen ist es möglich, diese iterativen Optimierungsversuche an juvenile (ca. 6 Monate) Spenderschweine zu adaptieren und in reduziertem Umfang bei den erfolgversprechenden Enzymkombinationen zu wiederholen.

Gleiches gilt auch für die Optimierungsversuche mit NB-1 Kollagenase bei der humanen Isolierungsprozedur. Unter praxisnaher Berücksichtigung der altersbedingten Spenderfaktoren, werden für Pankreasspender zwei Altersklassen gebildet, die bisher gewonnene Erfahrungen reflektieren: < 25 Jahre, ≥ 25 Jahre. Das oben angegebene Titrationsschema für neutrale Protease sollte für humane Pankreata adaptiert und bei zwei zuvor empirisch zu ermittelnden Kollagenasekonzentrationen (z. B. 4 mg/g und 5 mg/g Organ) durchgeführt werden:

| | Spender: | NB-1 Kollagenase: | | | |
|---|---|---|---|---|---|
| | | < 25 Jahre | | ≥ 25 Jahre | |
| 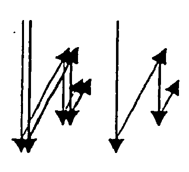 | Neutr. Protease: | 4 mg/g | 5 mg/g | 4 mg/g | 5 mg/g |
| | 0.5 DMC-U/g | | | | |
| | 1.0 DMC-U/g | | | | |
| | 1.5 DMC-U/g | | | | |
| | 2.0 DMC-U/g | | | | |
| | 2.5 DMC-U/g | | | | |

Die vermutlich effektivste Kombination an NB-1 Kollagenase und neutraler Protease wird für jede Altersklasse an 6 Organen getestet und die isolierten Inseln einer in vitro und in vivo Qualitätskontrolle (s. o.) unterzogen.

Durch die Verwendung von neutraler Protease mit gereinigter oder angereinigter Kollagenase mit definiertem niedrigen Gehalt an neutraler Protease ist eine individuelle Dosierung möglich. Eine Präparation des Enzymgemisches erfolgt in Bezug auf Units statt Masse, da die spezifische Aktivität sehr unterschiedlich ist.

Anpassung der neutralen Proteaseaktivität fördert erfolgreich die Isolierung von Inselzellen vom Schwein nach kalter Langzeitlagerung

### Hintergrund:

Die enzymatische Dissoziierung von Pankreasgewebe durch Kollagenase stellt einen entscheidenden Schritt im Verfahren der Isolierung von Inselzellen dar. Die Variabilität des pankreatitischen Kollagens bei verschiedenen Arten führte zur Herstellung spezifischer Enzymmischungen. Die derzeit verfügbaren Enzymmischungen bieten jedoch nicht die Möglichkeit, einzelne Bestandteile an Hand individueller Spendervariablen, z. B. der Kalt-Ischämiezeit, zu dosieren. Wir vermuten, dass die individuelle Anpassung der neutralen Proteaseaktivität als kritischer Faktor der enzymatischen Pankreasdigestion von Nutzen sein könnte, um die erfolgreiche Freisetzung von Inselzellen zu erleichtern. Um diese Hypothese zu beweisen, wurden Bauchspeicheldrüsen von Schweinen einer kalten Langzeitlagerung (6,5 Stunden) unterzogen, bevor die Dissoziierung mittels NB-8-Kollagenase von Serva vorgenommen wurde, die durch eine minimale Menge an neutraler Protease gekennzeichnet ist.

### Verfahren:

Nach der Entnahme wurden Bauchspeicheldrüsen von nicht mehr eingesetzten Zuchttieren intraduktal mit NB-8-Kollagenase, aufgelöst in einer kalten UW-Lösung, aufgebläht. Die endgültige kollagenolytische Aktivität betrug 4 FZ-U/g pankreatitischen Gewebes. Nach Vorexperimenten wurde neutrale Protease (Serva) an 0,6 bzw. 0,9 DMC-U/g bzw. 0,14 DMC-U/g für frisch beschaffte (n=5) bzw. gelagerte Bauchspeicheldrüsen (n=5) angepasst Inselzellen wurden durch modifizierte Digestion-Filtration isoliert und auf einem Cobe 2991 unter Nutzung von Ficoll-Natriumdiatrizoat gereinigt. Die Qualitätskontrolle umfasste die Trypan-blau-Ausschlussprobe und die statische Glukoseinkubation (2,8 gegenüber 20 mM Glukose). Außerdem wurde die Mitochondrienaktivität an Hand der Formazanproduktion geschätzt und bei 490 nm unter Verwendung einer Tetrazolverbindung (MTS) ermittelt und als willkürliche Einheiten (AU) pro mg Protein ausgedrückt.

### Ergebnisse:

Die Gesamtdigestionszeit bei lange gelagerten Bauchspeicheldrüsen war im Vergleich zu frisch beschaffter Bauchspeicheldrüsen deutlich verkürzt (48±3 gegenüber 63±3, P < 0,05). Hinsichtlich des Pankreasgewichts und der Menge digestierten Gewebes wurden keine Unterschiede festgestellt. Nach der Reinigung von Inselzellen, die mit 0,14 bzw. 0,9 DMC-U/g isoliert wurden, waren bezüglich der endgültigen Reinheit (> 95 %), der Inselzellenteilung (> 95 %) und der Rückgewinnung von Inselzellgewebe (73±9 gegenüber 72±16) keine bedeutsamen Unterschiede festgestellt. Nach der Langzeitlagerung war allerdings die Ausbeute an Inselzellen im Vergleich zu frisch isolierten Präparationen (146,000±43,000 gegenüber 212,000±52,000 IEQ, NS) leicht, aber nicht signifikant verringert. Die Qualitätskontrolle ergab eine ausgezeichnete Inselzellenlebensfähigkeit (99,9±0,1 gegenüber 99,4±0,3 %, NS), bewahrten intrazellulären Insulingehalt (1720±96 gegenüber 1760±280 µU/IEQ, NS) sowie einen vergleichbaren Glukosestimulationsindex (1,7±0,2 gegenüber 1,5±0,1, NS) nach der Digestion mit 0,14 bzw. 0,6 bzw. 0,9 DMC-U/g. Die Mitochondrienaktivität differierte zwischen den nach Langzeitlagerung isolierten Inselzellen (0,85±0,06 AU/mg) und frisch beschafften Zellen (0,90±0,11 AU/mg) nicht.

### Schlussfolgerungen:

Die vorliegende Studie demonstriert am Modell langzeitgelagerter Bauchspeicheldrüsen vom Schwein, dass die individuelle Dosierung einzelner Bestandteile einer Enzymmischung entsprechend den individuellen Spendervariablen nützlich ist, um die Freisetzung von Inselzellen zu erleichtern. Die individuelle Anpassung der neutralen Proteaseaktivität könnte auch für die Erwägung der warmen Ischämie, des Spenderalters und der fettigen oder faserigen Degeneration von Pankreasgewebe von Nutzen sein.

### Beispiel II

Bestimmung des optimalen Verhältnisses von neutraler Protease zu Kollagenase für die Isolierung von Langerhans-Inseln.

### Zielstellung:

Bestimmung des optimalen Verhältnisses von neutraler Protease/Kollagenase (NPCR) in der Enzymmischung, die für die Isolierung von Langerhans-Inseln eingesetzt wird. Serva-Kollagenasemischungen mit unterschiedlichen NPCR werden miteinander und mit Liberase und Kollagenase Typ V als Kontrollen bei einem Rattenmodell verglichen.

### Hintergrund:

Liberase ist gegenwärtig die universell eingesetzte Enzymmischung für die Isolierung von Human-Inseln. Ihre Entwicklung war eine definitive Verbesserung gegenüber Rohkollagenasen dergestalt, dass sie gereinigt war, frei von Endotoxin und wenig ihrer Unterschiedlichkeit von Charge zu Charge bei der enzymatischen Aktivität zeigte. Da die Ergebnisse der Inseltransplantation jedoch schnell voranschreiten, ergibt sich die Notwendigkeit der weiteren Verbesserung in der Gestaltung und Standardisierung der Enzymmischungen, die auf dem Markt erhältlich sind. Es gibt zum Beispiel keine Standardisierung der freigesetzten Liberase-Chargen hinsichtlich von Aktivitäten pro Gramm des Produktes der verschiedenen enzymatischen Komponenten. Es gibt daher eine beträchtliche Unterschiedlichkeit von Charge zu Charge bei der Kollagenase-Aktivität, der Aktivität der neutralen Protease und beim NPCR, wie dies in den Analysezertifikaten des Herstellers ausgewiesen wird. Aus diesem Grunde ist das optimale NPCR für die Inselisolierung nicht bekannt. Es ist ebenfalls vorstellbar, dass für jede Pankreas ein angepaßtes NPCR erforderlich sein könnte, entsprechend ihrer Struktur, dem Alter des Spenders, der Ischämiezeit. Ein neues Enzym, das auf dem Markt erhältlich ist, ist Serva Kollagenase, mit der neutralen Protease in separaten Phiolen verpackt. Dies gestattet eine maßgeschneiderte Mischung der zwei Komponenten, um ein vorherbestimmtes Verhältnis zu erzielen, anstatt, dass die Isolierung mit einem Verhältnis durchgeführt wird, das vom Hersteller vorher festgelegt wurde und von Charge zu Charge variiert. Am Ratten-Modell ist zu bestimmen, welches das optimale NPCR für Inselisolierung ist.

### Verfahrensweisen:

Sieben Gruppen von Lewis-Ratten werden als Insel-Spender genutzt werden. In jeder Gruppe werden sich 18 Ratten befinden. Die Gruppen werden wie folgt bestimmt werden:
- Gruppe 1:: Inselisolierung mit reiner Serva-Kollagenase, ohne neutrale Protease (NPCR = 0 % w/w)
- Gruppe 2:: Inselisolierung mit Serva-Kollagenase, mit einem NPCR von 3 % w/w
- Gruppe 3:: Inselisolierung mit Serva-Kollagenase, mit einem NPCR von 6 % w/w
- Gruppe 4:: Inselisolierung mit Serva-Kollagenase, mit einem NPCR von 12 % w/w
- Gruppe 5:: Inselisolierung mit Serva-Kollagenase, mit einem NPCR von 24 % w/w
- Gruppe 6:: Inselisolierung erfolgt mit Liberase RI
- Gruppe 7:: Inselisolierung erfolgt mit Kollagenase Typ V (Sigma).

### Beispiel IIA

Die Rolle neutraler Protease während der Inselisolierung, bewertet mit einem neuen Enzympräparat

### Zielstellung:

Für die Inselisolierung stehen mehrere Enzympräparate zur Verfügung. Eines derselben ist ein hochgereinigtes Produkt mit separaten Komponenten (Kollagenase und neutrale Protease), welches die Adaptation der neutralen Protease-Konzentration für die Inselisolierung gestattet. Bei dieser Studie bewerten wir die Rolle neutraler Protease während der Inselisolierung.

### Verfahren:

Sieben Gruppen von Sprague-Daley Ratten (18 Ratten pro Gruppe) wurden für die standardisierte Inselisolierung gemäß dem Enzymtyp gebildet. Wir setzten bei Gruppe I Kollagenase Typ XI bei 2mg/ml ein (Sigma Chemical), bei Gruppe II Liberase bei 0,6mg/ml (Roche) und in den Gruppen III bis VII Kollagenase NB1 0,6mg/ml (Serva Elektrophorese). Bei Gruppe III wurde reine Kollagenase NB 1 eingesetzt. Bei den Gruppen IV, V, VI und VII wurde eine Konzentration von 7,5, 15, 22 und/oder 30 µg/ml neutrale Protease (NP)hinzugefügt. Die Ergebnisse wurden bewertet hinsichtlich von Inselausbeuten, Apoptose (Zelltoddetektion ELISA, Roche und Tunel-Färbung), von Zytokinen (IL-1β, IL-6, TNFα, IFNγ) Sekretionen (Quantikine M, R&D System), Insulin-Sekretion (statische Inkubation) und Insel-Morphologie (Histologie und Immunhistologie).

### Ergebnisse:

Der Endotoxin-Gehalt der Enzymlösung betrug für die Gruppen I und II 173 + 22 und 120 + 11 EU/ml. Bei den Gruppen III bis VII erhöhte er sich allmählich, gemäß der NP-Konzentration, von 0,4 auf 58 EU/ml (p<0,05). Die Inseläquivalent-(IE)-Ausbeuten pro Ratte beliefen sich auf 1367 + 522 und auf 1755 + 110 für Gruppe I und/oder II. Bei den Gruppen III bis VII, folgten die IE-Ausbeuten pro Ratte einer Gaußschen Normalverteilung gemäß der NP-Konzentration, mit einer höheren Ausbeute bei der Gruppe V, 1712 + 245, und einer niedrigeren für die Gruppe III (597 + 277) und die Gruppe VII (905 + 297) (p=0,05). Bei den Gruppen III bis VII wurde die Insel-Morphologie beeinflusst von der NP-Konzentration, mit einer abnehmenden Anzahl von gefangenen Inseln und einer steigenden Anzahl von fragmentierten Inseln, in dem Maße, wie sich der NP-Gehalt erhöhte. Die Erhöhung der NP-Konzentration war verbunden mit einer progressiven Schädigung des α-Zellrings der Insel, die bei der Gruppe VII vergleichbar war mit der Schädigung, die bei den Gruppen I und II beobachtet wurde. Die Freisetzung von Zytokinen (IL-1β, IL-6, TNFα, IFNγ) war niedriger in den Gruppen III bis VII im Vergleich zu den Gruppen I und II, sie wurde jedoch nicht von der NP-Konzentration beeinflusst. Insel-Zellnekrose und Apoptose werden statistisch signifikant geringer in den Gruppen III bis VII im Vergleich zu den Gruppen I und II, sie wurden jedoch nicht von der NP-Konzentration beeinflusst. Die mittleren Stimultionsindexe der Insulinsekretion waren 2,96 für die Gruppe I, 5,17 für die Gruppe II, und sie liegen zwischen 5,25 und 5,43 für die Gruppen III bis VII.

### Schlussfolgerung:

Neutrale Protease ist ein entscheidender Zusatz zu Kollagenase für die Inselisolierung hinsichtlich von Inselausbeuten. Die Serva-Kollagenase mit einer geeigneten Konzentration neutraler Protease ist vergleichbar mit der Liberase hinsichtlich von Inselausbeuten und Funktion. Sie ist jedoch verbunden mit niedriger Freisetzung von Inselzellen-Zytokinen und mit Apoptose, was zu einer verbesserten Insel-Morphologie führt.

### Beispiel III

Erfolgreiche Langzeiterhaltung des Pankreas mittels des Zweischichtverfahrens zur nachfolgenden Isolierung von Inselzellen vom Schwein

### Hintergrund:

Die besondere Empfindlichkeit von Inselzellen vom Schwein verhinderte die erfolgreiche Isolierung aus Bauchspeicheldrüsen nach kalter Langzeitlagerung. Zur erfolgreichen Isolierung menschlicher Inselzellen wurde kürzlich die Sauerstoffanlagerung an Pankreasgewebe mittels des Zweischichtverfahrens (TLM) während der kalten Lagerung entwickelt. Bislang stehen noch keine Daten über die Langzeitkonservierung von Schweine-Bauchspeicheldrüsen mittels TLM zur Verfügung. Deshalb war die vorliegende Studie darauf gerichtet, die Effizienz des TLM für die erfolgreiche Isolierung von Inselzellen aus Schweine-Bauchspeicheldrüsen untersucht, die einer TLM-Konservierung über 6,5 Stunden (n=5) im Vergleich zu frisch beschafften Bauchspeicheldrüsen (n=5) unterzogen wurden.

### Verfahren:

Unmittelbar nach der Entnahme wurden Bauchspeicheldrüsen von nicht mehr eingesetzten Zuchttieren intraduktal mit Kollagenase (Serva), aufgelöst in einer kalten UW-Lösung mit einer Konzentration von 4 mg pro g Bauchspeicheldrüsengewebe, aufgebläht. Inselzellen wurden durch modifizierte Digestion-Filtration isoliert und auf einem Cobe 2991 unter Nutzung von Ficoll-Natriumdiatrizoat gereinigt. Die Qualitätskontrolle umfasste die Trypan-blau-Ausschlussprobe und die statische Glukoseinkubation (2,8 gegenüber 20 mM Glukose). Außerdem wurde die Mitochondrienaktivität an Hand der Formazanproduktion geschätzt und bei 490 nm unter Verwendung einer Tetrazolverbindung (MTS) ermittelt und als willkürliche Einheiten (AU) pro mg Protein ausgedrückt.

### Ergebnisse:

Nach der Reinigung wurden keine signifikanten Unterschiede zwischen frisch beschafften Bauchspeicheldrüsen und TLM-konservierten Bauchspeicheldrüsen hinsichtlich der endgültigen Reinheit (> 95 %), der Ausbeute (2440±580 gegenüber 1800±250 IEQ/g), der Lebensfähigkeit (99,4±0,3 gegenüber 100,0±0,0) und des Glukosestimulationsindex (1,73±0,19 gegenüber 1,48±0,14, NS) von Inselzellen festgestellt. Der intrazelluläre Insulingehalt war nach der TLM-Konservierung im Vergleich zur Frischbeschaffung erhöht (2250±110 gegenüber 1760±280 µU/IEQ, NS). Im Vergleich zu frisch isolierten Inselzellen wurde die Mitochondrienaktivität durch das TLM während der Langzeitlagerung bewahrt (0,96±0,15 gegenüber 1,01±0,17 AU/mg, NS).

### Schlussfolgerungen:

Die vorliegende Studie demonstriert die Effizienz des TLM für die Langzeitkonservierung von Schweine-Bauchspeicheldrüsen vor der erfolgreichen Isolierung von Inselzellen.

### Beispiel IV

Umfasst die Lagerung des Gewebes (Schweinepankreas) mit der TWO-layer Methode auf Perfluorhydrocarbon, Gewebedissoziation durch individuelle Zugabe von neutraler Protease.

Perfluorhydrocarbon hat eine hohe Sauerstoff-Bindungsfähigkeit. Dadurch wird das gelagerte Gewebe besser mit Sauerstoff versorgt, so dass der Stoffwechsel besser aufrechterhalten werden kann und das Gewebe die Lagerung von einigen Stunden besser übersteht. Für die Isolierung von Zellen wurde die verdauende Enzymlösung entweder vor der Lagerung (TLM preloaded) oder nach der Lagerung (TLM postloaded) zugegeben. Wiederum wichtig war die Variationsmöglichkeit der Menge der zugegebenen neutralen Protease (bei gleichbleibender Kollagenase-Konzentration von 4 PZ-U/g Organ): preload geringere Menge (0,1 DMC-U/g Organ, da durch die Lagerung eine längere Verdauzeit gegeben ist) und postload höhere Menge (0,9 DMC-U/g Organ).

Dabei Optimierung der Zellisolierungsergebnisse durch Variation der neutralen Protease und der Lagerungsmethode.

Daten in der Tabelle I:
Dies sind die detaillierten Daten bezüglich Ausbeute an Inselzell-Äquivalenten, Viabilität, Unversehrtheit der Inselzellen.
Die Daten zeigen, dass man unabhängig von Lagerung (2=TLM-preloaded (4 PZ-U; 0,1 DMC-U); 4=UW-preloaded (4 PZ-U; 0,1 DMC-U); 3=TLM-postloaded (4 PZ-U; 0,9 DMC-U)) oder Nicht-Lagerung (6=Unstored (4 PZ-U; 0,9 DMC-U)) vergleichbar gute Ergebnisse im Hinblick auf Ausbeute an Inselzell-Äquivalenten, Viabilität, Unversehrtheit der Inselzellen bzw. Inselzellaggregate erzielen kann, wenn die Menge an neutraler Protease entsprechend angepasst wird.

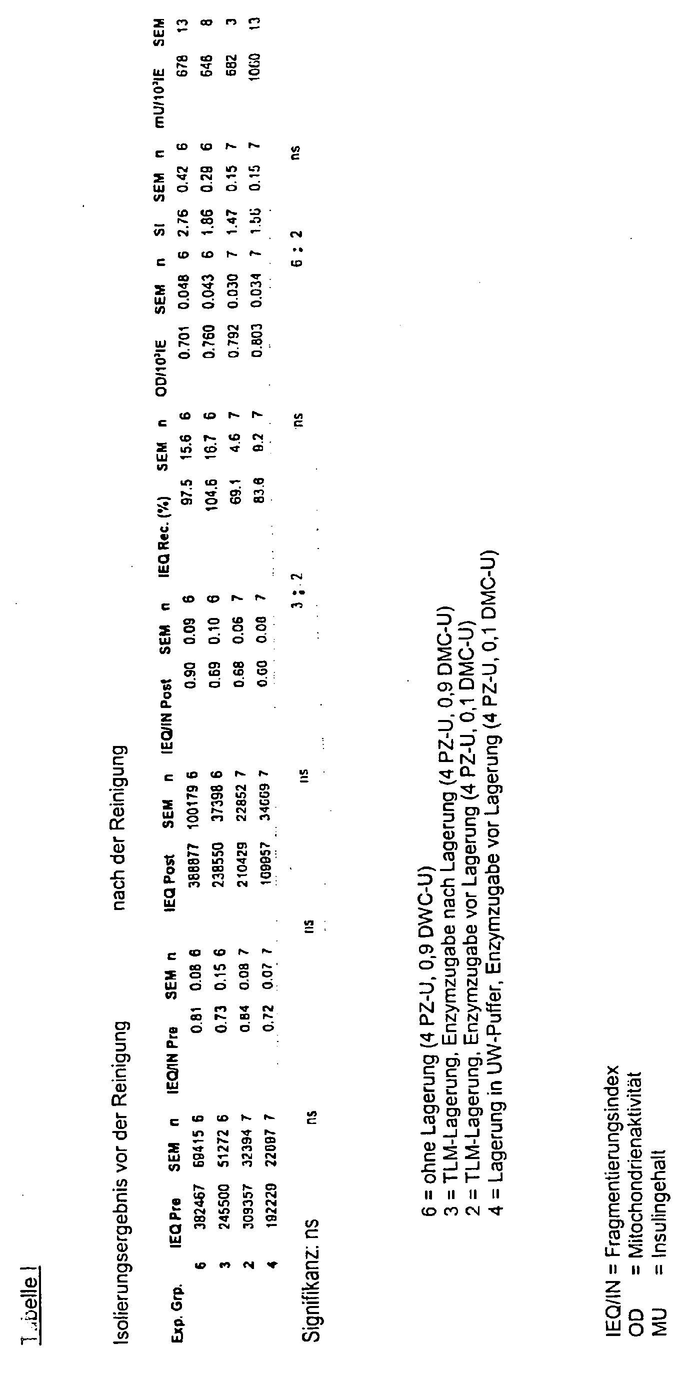

## Patentansprüche

1. Verfahren zur Isolierung von Inselzellen aus Pankreasgewebe, umfassend die Schritte:
(a) Bereitstellen (i) einer separaten Komponente A aus einer nicht in einer Kollagenase-Enzympräparation enthaltenen Protease (NP) aus *Clostridium histolyticum* in einer in einem Bereich von 4 bis 8 mg/g Pankreasgewebe liegenden Menge und (ii) einer separaten Komponente B aus Kollagenase bzw. aus einer Kollagenase-Enzympräparation in einer in einem Bereich von 0,2 bis 0,6 DMC-U/g Pankreasgewebe liegenden Menge;
(b) Mischen der Komponente A und der Komponente B unmittelbar vor der Zugabe zu dem Pankreasgewebe, wobei das Verhältnis der Komponente A zu Komponente B in Abhängigkeit von der Art der Spezies, aus der das Pankreasgewebe stammt, dem Zustand des Pankreasgewebes, den Lagerungsbedingungen des Pankreasgewebes und/oder der Menge des Pankreasgewebes bestimmt wird, wobei der Gewichtsanteil von Komponente A an der erhaltenen Mischung 3, 6, 12 oder 24 % beträgt;
(c) Zugeben der gemäß Schritt (b) zubereiteten Mischung zu dem Pankreasgewebe; und
(d) Isolation der Inselzellen.

2. Verfahren nach Anspruch 1
**dadurch gekennzeichnet,**
**dass** Schritt (b) die Schritte
(b1) Bestimmen der Menge der Komponente B in Abhängigkeit der Menge des Pankreasgewebes; und
(b2) Zugabe einer vorgegebenen Menge der Komponente A zu der Komponente B durch Titration
umfasst.

3. Verfahren nach Anspruch 1 und/oder 2,
**dadurch gekennzeichnet,**
**dass** nach Zugabe des Gemisches in Schritt (c) eine Lagerung der so erhaltenen Präparation erfolgt.

4. Verfahren nach Anspruch 3,
**dadurch gekennzeichnet,**
**dass** die Lagerung nach der TWO-Layer-Methode mit Perfluorhydrocarbon vorgenommen wird.

5. Verfahren nach Anspruch 4,
**dadurch gekennzeichnet,**
**dass** in Schritt (c) die Mischung vor der Lagerung des Pankreasgewebes nach der TWO-Layer-Methode zu dem Pankreasgewebe zugegeben wird.

6. Verfahren nach Anspruch 4,
**dadurch gekennzeichnet,**
**dass** in Schritt (c) die Mischung nach der Lagerung des Pankreasgewebes gemäß der TWO-Layer-Methode zu dem Pankreasgewebe zugegeben wird.

7. Verfahren nach einem der Ansprüche 4 bis 6,
**dadurch gekennzeichnet,**
**dass** der Anteil der Komponente A an dem in Schritt (b) erhaltenen Gemisch bei der Zugabe der Mischung vor der Lagerung des Pankreasgewebes gemäß der TWO-Layer-Methode zu dem Pankreasgewebe geringer als bei der Zugabe der Mischung nach der Lagerung des Pankreasgewebes gemäß der TWO-Layer-Methode zu dem Pankreasgewebe ist.

8. Verfahren zur Herstellung eines Produktes aus neutraler Protease (NP) und einer Kollagenase für die Isolierung von Inselzellen aus Pankreasgewebe, umfassend die Schritte:
(a) Bereitstellen (i) einer separaten Komponente A aus einer nicht in einer Kollagenase-Enzympräparation enthaltenen Protease (NP) aus *Clostridium histolyticum* in einer in einem Bereich von 4 bis 8 mg/g Pankreasgewebe liegenden Menge und (ii) einer separaten Komponente B aus Kollagenase bzw. aus einer Kollagenase-Enzympräparation in einer in einem Bereich von 0,2 bis 0,6 DMC-U/g Pankreasgewebe liegenden Menge; und
(b) Mischen der Komponente A und der Komponente B unmittelbar vor der Zugabe zu dem Pankreasgewebe, wobei das Verhältnis der Komponente A zu Komponente B in Abhängigkeit von der Art der Spezies, aus der das Pankreasgewebe stammt, dem Zustand des Pankreasgewebes, den Lagerungsbedingungen des Pankreasgewebes und/oder der Menge des Pankreasgewebes bestimmt wird, wobei der Gewichtsanteil von Komponente A an der erhaltenen Mischung 3, 6, 12 oder 24 % beträgt.

9. Verfahren nach Anspruch 8
**dadurch gekennzeichnet,**
**dass** Schritt (b) die Schritte
(b1) Bestimmen der Menge der Komponente B in Abhängigkeit der Menge des Pankreasgewebes; und
(b2) Zugabe einer vorgegebenen Menge der Komponente A zu der Komponente B durch Titration
umfasst.

10. Verwendung eines nach Anspruchs 8 oder Anspruch 9 hergestellten Produktes aus neutraler Protease (NP) und einer Kollagenase für die Isolierung von Inselzellen aus Pankreasgewebe zur Verbesserung der Isolierungsergebnisse im Hinblick auf Ausbeute, Viabilität und Unversehrtheit der Zellen.

## Claims

1. A method for the isolation of islet cells from pancreatic tissue, comprising the steps of:
(a) providing (i) a separate component A from a protease (NP) from *Clostridium histolyticum* not contained in a collagenase enzyme preparation in an amount within a range from 4 to 8 mg/g of pancreatic tissue, and (ii) a separate component B from collagenase or from a collagenase enzyme preparation, respectively, in an amount within a range from 0.2 to 0.6 DMC-U/g of pancreatic tissue;
(b) mixing component A and component B immediately before the addition to the pancreatic tissue, wherein the ratio of component A to component B is determined depending on the type of species, from which the pancreatic tissue originates, the condition of the pancreatic tissue, the storage conditions of the pancreatic tissue and/or the amount of the pancreatic tissue, wherein the weight proportion of component A in the mixture obtained is 3, 6, 12 or 24 %;
(c) adding the mixture prepared according to step (b) to the pancreatic tissue; and
(d) isolating the islet cells.

2. The method according to claim 1, **characterized in that** step (b) comprises the steps of:
(b1) determining said amount of component B depending on the amount of the pancreatic tissue; and
(b2) adding a predetermined amount of component A to component B by titration.

3. The method according to claim 1 and/or 2, **characterized in that** following addition of said mixture in step (c) the preparation thus obtained is stored.

4. The method according to claim 3, **characterized in that** said storage takes place according to the TWO layer method with perfluorohydrocarbon.

5. The method according to claim 4, **characterized in that** in step (c) said mixture is added to the pancreatic tissue before said storage of the pancreatic tissue according to the TWO layer method.

6. The method according to claim 4, **characterized in that** in step (c) said mixture is added to the pancreatic tissue after said storage of the pancreatic tissue according to the TWO layer method.

7. The method according to any of claims 4 to 6, **characterized in that** the proportion of said component A in the mixture obtained in step (b) is lower upon addition of said mixture to the pancreatic tissue before said storage of the pancreatic tissue according to the TWO layer method than upon addition of said mixture to the pancreatic tissue after said storage of the pancreatic tissue according to the TWO layer method.

8. A method for producing a product from neutral protease (NP) and a collagenase for the isolation of islet cells from pancreatic tissue, comprising the steps of:
(a) providing (i) a separate component A from a protease (NP) from *Clostridium histolyticum* not contained in a collagenase enzyme preparation in an amount within a range from 4 to 8 mg/g of pancreatic tissue, and (ii) a separate component B from collagenase or from a collagenase enzyme preparation, respectively, in an amount within a range from 0.2 to 0.6 DMC-U/g of pancreatic tissue; and
(b) mixing component A and component B immediately before the addition to the pancreatic tissue, wherein the ratio of component A to component B is determined depending on the type of species, from which the pancreatic tissue originates, the condition of the pancreatic tissue, the storage conditions of the pancreatic tissue and/or the amount of the pancreatic tissue, wherein the weight proportion of component A in the mixture obtained is 3, 6, 12 or 24 %.

9. The method according to claim 8, **characterized in that** step (b) comprises the steps of:
(b1) determining said amount of component B depending on the amount of the pancreatic tissue; and
(b2) adding a predetermined amount of component A to component B by titration.

10. Use of a product produced from neutral protease (NP) and a collagenase according to claim 8 or claim 9 for the isolation of islet cells from pancreatic tissue for improvement of the isolation results in respect of yield, viability and intactness of the cells.

## Revendications

1. Procédé d'isolation de cellules insulaires de tissu pancréatique, comprenant les étapes consistant à :
(a) se procurer (i) un composant séparé A issu d'une protéase (NP) de *Clostridie historiquement* non contenue dans une préparation enzymatique à la collagénose en une quantité située dans une plage de 4 à 8 mg/g de tissu pancréatique et un composant séparé B issu de collagénose ou d'une préparation enzymatique à la collagénose en une quantité située dans une plage de 0,2 à 0,6 DMC-U/g de tissu pancréatique ;
(b) mélanger le composant A et le composant B immédiatement avant l'ajout dans le tissu pancréatique, le rapport entre le composant A et le composant B étant défini en fonction du type d'espèce dont provient le tissu pancréatique, de l'état du tissu pancréatique, des conditions de stockage du tissu pancréatique et/ou de la quantité de tissu pancréatique, la proportion en poids de composant A dans le mélange obtenu étant de 3, 6, 12 ou 24 % ;
(c) ajouter le mélange préparé suivant l'étape (b) dans le tissu pancréatique ; et
(d) isoler les cellules insulaires.

2. Procédé selon la revendication 1, **caractérisé en ce que** l'étape (b) comprend les étapes consistant à :
(b1) déterminer la quantité de composant B en fonction de la quantité de tissu pancréatique ; et
(b2) ajouter une quantité prédéfinie de composant A au composant B par titrage.

3. Procédé selon la revendication 1 et/ou 2, **caractérisé en ce que,** après ajout du mélange en étape (c), on procède à un stockage de la préparation ainsi obtenue.

4. Procédé selon la revendication 3, **caractérisé en ce que** le stockage se fait suivant le procédé à 2 couches avec du perfluorocarbure.

5. Procédé selon la revendication 4, **caractérisé en ce que,** dans l'étape (c), avant le stockage du tissu pancréatique suivant le procédé à 2 couches, on incorpore le mélange au tissu pancréatique.

6. Procédé selon la revendication 4, **caractérisé en ce que,** dans l'étape (c), après le stockage du tissu pancréatique suivant le procédé à 2 couches, on incorpore le mélange au tissu pancréatique.

7. Procédé selon une des revendications 4 à 6, **caractérisé en ce que** la proportion de composant A dans le mélange obtenu en étape (b) est plus faible en cas d'ajout du mélange avant le stockage du tissu pancréatique suivant le procédé à 2 couches qu'en cas d'ajout du mélange après le stockage du tissu pancréatique suivant le procédé à 2 couches.

8. Procédé de préparation d'un produit issu de protéase neutre (NP) et de collagénose pour l'isolation de cellules insulaires de tissu pancréatique, comprenant les étapes consistant à :
(a) se procurer (i) un composant séparé A issu d'une protéase (NP) de *Clostridie historiquement* non contenue dans une préparation enzymatique à la collagénose en une quantité située dans une plage de 4 à 8 mg/g de tissu pancréatique et un composant séparé B issu de collagénose ou d'une préparation enzymatique à la collagénose en une quantité située dans une plage de 0,2 à 0,6 DMC-U/g de tissu pancréatique ;
(b) mélanger le composant A et le composant B immédiatement avant l'ajout dans le tissu pancréatique, le rapport entre le composant A et le composant B étant défini en fonction du type d'espèce dont provient le tissu pancréatique, de l'état du tissu pancréatique, des conditions de stockage du tissu pancréatique et/ou de la quantité de tissu pancréatique, la proportion en poids de composant A dans le mélange obtenu étant de 3, 6, 12 ou 24 %.

9. Procédé selon la revendication 8, **caractérisé en ce que** l'étape (b) comprend les étapes consistant à :
(b1) déterminer la quantité de composant B en fonction de la quantité de tissu pancréatique ; et
(b2) ajouter une quantité prédéfinie de composant A au composant B par titrage.

10. Utilisation d'un produit préparé selon la revendication 8 ou la revendication 9 à partir de protéase neutre (NP) et d'une collagénose pour l'isolation de cellules insulaires de tissu pancréatique pour améliorer les résultats d'isolation du point de vue du rendement, de la viabilité et de la pureté des cellules.
